# EUROPEAN PATENT APPLICATION

(11) **EP 4 413 858 A1**
(43) Date of publication of application: **14.08.2024**
(21) Application number: 24156155.4
(22) Date of filing: 06.02.2024
(51) Int. Cl.: A01N 1/00, A01N 25/02, A61K 9/00, A61K 9/127, A61K 31/704

(54) **SOLUTION FOR PRESERVING EXTRACELLULAR VESICLES/EXOSOMES, METHOD FOR PRESERVING THE SAME, AND MIXED SOLUTION THEREOF**

(30) Priority: 10.02.2023 US 202363484232 P
(71) Applicant: China Medical University, Taichung City 406040 (TW)
(72) Inventor: CHEN, Yi-Wen, 404 Taichung City (TW); SHIE, Ming-You, 404 Taichung City (TW); CHEN, Yen, 540009 Nantou City, Nantou County (TW); CHO, Der-Yang, 404 Taichung City (TW); CHIU, Shao-Chih, 404 Taichung City (TW); LIN, Yen-Hong, 407215 Taichung City (TW); KAN, Kai-Wen, 404 Taichung City (TW)
(74) Representative: Cabinet Chaillot

(57) **Abstract**

A solution for preserving extracellular vesicles/exosomes has a carrier solvent selected from a group consisting of polysorbate 80, sucrose, and polyethylene glycol 3350/4000; and a stabilizer selected from a group consisting of salts, amino acids, and amino acid salts; wherein the pH of the preservation solution ranges from 5 to 7.4.

## Description

Preservation solution, in particular solution formulation and method capable of long-term preservation of extracellular vesicles/exosomes in a frozen environment.

Due to the challenge of preservation and susceptibility to degradation after purification, the extracellular vesicles/exosomes are often purified according to the amount required for each application. Subsequent modification or drug conjugation, if implemented, facilitates to extend the overall process duration and presents challenges to progress towards industrial development.

To overcome the challenges of difficulty in preservation and susceptibility to degradation of the extracellular vesicles/exosomes after purification, the present invention provides a solution for preserving the extracellular vesicles/exosomes comprising: a carrier solvent selected from the group consisting of polysorbate 80, sucrose, and polyethylene glycol 3350/4000 (PEG-3350/4000); and a stabilizer selected from the group consisting of salts, amino acids, and amino acid salts. The pH of the preservation solution is in the range of 5 to 7.4.

Wherein, the salt is a sodium acetate or a sodium chloride, and the weight percent concentration of the salt is in the range of 0.1 to 2 of the total volume of the preservation solution.

Wherein, the amino acids are selected from the group consisting of histidine, arginine, and glycine, and the weight percent concentration of the amino acid is in the range of 0.5 to 3 of the total volume of the preservation solution.

Wherein, the amino acid salts are selected from the group consisting of histidine hydrochloride, arginine hydrochloride, and glycine hydrochloride, and the weight percent concentration of the amino acid salt is in the range of 0.05 to 0.25 of the total volume of the preservation solution.

Wherein, the weight percent concentration of polysorbate 80 is in the range of 0.01 to 0.03 of the total volume of the preservation solution; the weight percent concentration of sucrose is in the range of 5 to 10 of the total volume of the preservation solution; and the weight percent concentration of polyethylene glycol 3350/4000 (PEG-3350/4000) is in the range of 1 to 5 of the total volume of the preservation solution.

The present invention also provides a method for preserving the extracellular vesicles/exosomes. The steps include:
adding the extracellular vesicles/exosomes to the preservation solution to form a mixed preservation solution; and
gradually cooling the mixed preservation solution and placing it in a frozen preservation environment.

Wherein, the temperature of the frozen preservation environment may be -20 degrees Celsius or -80 degrees Celsius.

Wherein, after forming the mixed preservation solution, drying to form a dried preservation sample, and then placing the dried preservation sample in the frozen preservation environment.

The present invention further provides a mixed solution, comprising the preservation solution and the extracellular vesicles/exosomes.

Wherein, the extracellular vesicles/exosomes in the mixed preservation solution are in the range of 10¹¹ to 10¹³ cells per milliliter.

Wherein, the extracellular vesicles/exosomes are modified vesicles carrying a specific protein.

Wherein, the extracellular vesicles/exosomes are carried with a drug.

The present invention utilizes the selected carrier solvent and the stabilizer in the preservation solution to configure appropriate preservation conditions and pH according to the different types of extracellular vesicles/exosomes and to enable long-term preservation under frozen conditions.

Additionally, the preservation solution can preserve the extracellular vesicles/exosomes in various preservation environments, which contributes to the commercial development of the product.

Through the configuration of the preservative solution, the present invention enables frozen preservation based on specific liquid or dry preservation forms as needed to achieve long-term preservation of the extracellular vesicles/exosomes. Upon thawing and application, a certain reaction efficiency can be maintained, which contributes to research and application in the medical industry, and enhances time cost and commercial value.

The present invention will be further described below with reference to one of the preferred embodiments shown in the accompanying drawings, wherein:
FIG. 1 is a step flow chart of the first preferred embodiment of the method of preparation provided by the present invention;
FIG. 2 is a concentration test chart of extracellular vesicles of the first preferred embodiment provided by the present invention;
FIG. 3 is an expression chart of marker proteins on extracellular vesicles of the first preferred embodiment provided by the present invention;
FIG. 4 is a step flow chart of the second preferred embodiment of the method of preparation provided by the present invention;
FIG. 5 is a concentration test chart of extracellular vesicles of the second preferred embodiment provided by the present invention;
FIG. 6 is an expression chart of marker proteins on extracellular vesicles of the second preferred embodiment provided by the present invention;
FIG. 7 is a size analysis chart of extracellular vesicles of the second preferred embodiment provided by the present invention;
FIG. 8 is a drug-carrying analysis chart of extracellular vesicles of the preferred embodiment provided by the present invention;
FIG. 9 is a first test chart of the cytotoxic effect of extracellular vesicles of the preferred embodiment provided by the present invention;
FIG. 10 is a second test chart of the cytotoxic effect of extracellular vesicles of the preferred embodiment provided by the present invention;
FIG. 11 is a concentration test chart of long-term frozen extracellular vesicles of the preferred embodiment provided by the present invention;
FIG. 12 is a first test chart of the cytotoxic effect of long-term frozen extracellular vesicles of the preferred embodiment provided by the present invention; and

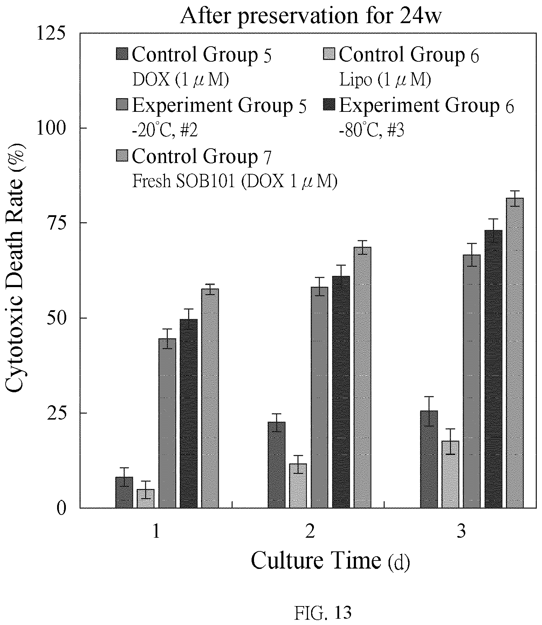

FIG. 13 is a second test chart of the cytotoxic effect of long-term frozen extracellular vesicles of the preferred embodiment provided by the present invention.

The present invention provides a preservation solution for preserving extracellular vesicles/exosomes comprising water, a carrier solvent, and a stabilizer, wherein the pH of the preservation solution is in the range of 5 to 7.4. The carrier solvent may be selected from the group consisting of polysorbate 80 (Tween 80), sucrose, and polyethylene glycol 3350/4000 (PEG-3350/4000). The stabilizer is used to stabilize the biological activity of the extracellular vesicles/exosomes mixed in the preservation solution to adapt to different preservation environments.

Preferably, the stabilizer includes amino acids or salts composed of amino acids. More preferably, the stabilizer is selected from the group consisting of histidine, arginine, glycine, histidine hydrochloride, arginine hydrochloride, and glycine hydrochloride.

Preferably, the stabilizer includes the salts to stabilize the pH of the preservation solution. More preferably, the salt is a sodium acetate or a sodium chloride.

The weight percent concentration of polysorbate 80 in the preservation solution is in the range of 0.01% to 0.03% of the total volume; and the weight percent concentration of sucrose is in the range of 5% to 10% of the total volume.

The weight percent concentration of amino acids in the preservation solution is in the range of 0.5% to 3% of the total volume; and the weight percent concentration of amino acid salts is in the range of 0.05% to 0.25% of the total volume.

The amount of salts added to the preservation solution is adjusted according to the pH requirements of the preservation solution.

Furthermore, the present invention provides Embodiments 1, 2 and 3, each conducting a cryopreservation method to preserve the extracellular vesicles/exosomes. The cryopreservation may be in the form of liquid preservation or dry preservation. In this regard, commonly used phosphate buffered saline (PBS) and dimethyl sulfoxide (DMSO) solutions, which are widely employed for preserving the extracellular vesicles/exosomes, are used as Comparative Examples 1 and 2, respectively.

Using the concentration of extracellular vesicles/exosomes in each Comparative Example and Embodiment as a reference, and comparing the amount of extracellular vesicles in each Comparative Example and Embodiment by the Nanoparticle Tracking Analysis to serve as the residual concentration percentage of the extracellular vesicles/exosomes in each Comparative Example and Embodiment. Furthermore, the residual concentration percentage of the extracellular vesicles/exosomes in each Comparative Example and Embodiment is compared after cryopreservation to evaluate the degradation rate of the extracellular vesicles/exosomes. This verifies the preservation efficacy of the preservation solution in the cryopreservation.

In Embodiments 1, 2, and 3, the carrier solvent and stabilizer are each added to the preservation solution, the pH is adjusted to the appropriate range, and then water is replenished to achieve the final required volume of the preservation solution. The formulations for Embodiments 1, 2 and 3 are shown in Tables 1 to 3.

**Table 1:**

| Embodiment 1 | | Weight Percentage % |
|---|---|---|
| Carrier Solvent | Polysorbate 80 (Tween 80) | 0.01% |
| | Sucrose | 5% |
| Stabilizer | Sodium Acetate | 0.1% |

**Table 2:**

| Embodiment 2 | | Weight Percentage % |
|---|---|---|
| Carrier Solvent | Polyethylene Glycol 3350/4000 (PEG-3350/4000) | 1% |
| | Sucrose | 5% |
| Stabilizer | Histidine | 0.1% |
| | Arginine | 0.2% |
| | Glycine | 0.2% |

**Table 3:**

| Embodiment 3 | | Weight Percentage % |
|---|---|---|
| Carrier Solvent | Polysorbate 80 (Tween 80) | 0.01% |
| Stabilizer | Histidine Hydrochloride | 0.1% |
| | Glycine | 0.5% |
| | Sodium Chloride | 0.1% |

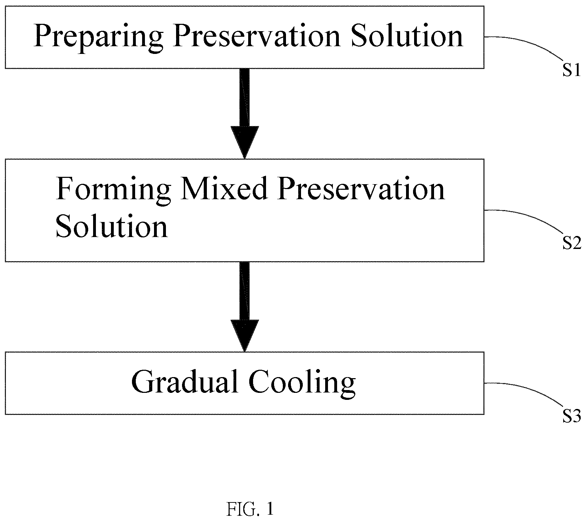

In Experiment 1, referring to FIGS. 1 to 3, the preservation solution is used to carry out the cryopreservation method for the extracellular vesicles/exosomes in the form of liquid preservation. The steps include:
S1, preparing the preservation solution. Based on the components in Tables 1 to 3, Embodiments 1, 2 and 3 are formed by mixing the components in Tables 1 to 3.
S2, forming a mixed preservation solution. The extracellular vesicles/exosomes are added to the preservation solution to form the mixed preservation solution.

Wherein, the concentration of the extracellular vesicles/exosomes in the mixed preservation solution, PBS, and DMSO is preferably between 10¹¹ and 10¹³ cells per milliliter.

In this embodiment, the extracellular vesicles are obtained using conventional extraction techniques. The extracellular vesicles/exosomes may be modified by known cell engineering techniques to carry specific proteins, and the extracellular vesicles/exosomes or the modified extracellular vesicles may also be engineered to encapsulate/carry drugs.

S3, gradual cooling. In a gradual cooling technique, the mixed preservation solution is gradually cooled and then placed in a frozen preservation environment. The cooling rate in this gradual cooling process ranges from 0.5°C/min to 2°C/min.

Wherein, the temperature of the frozen preservation environment may be -20 degrees Celsius or -80 degrees Celsius.

Taking -80 degrees Celsius as an example, the gradual cooling can be carried out at room temperature after preparation of the mixed preservation solution, followed by standing at -4 degrees Celsius and -20 degrees Celsius, and finally, preservation in the -80 degrees Celsius frozen environment.

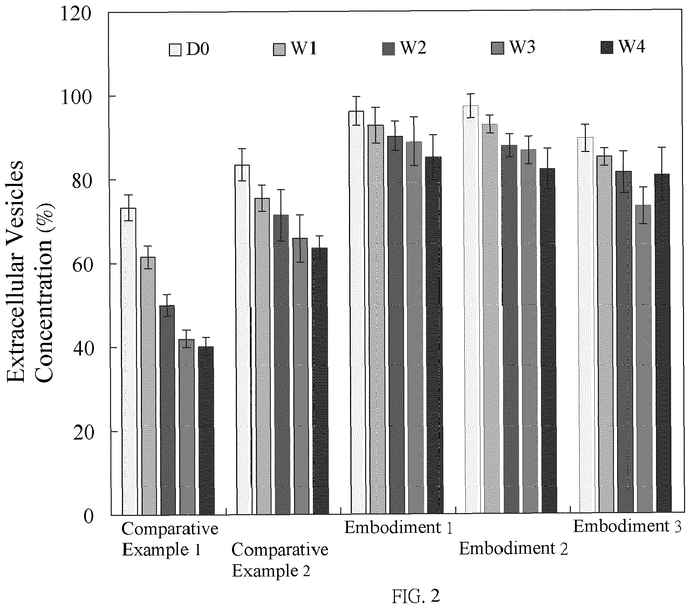

Next, please refer to FIG. 2, which illustrates a concentration test of the extracellular vesicles performed when the vesicles were mixed with Comparative Example 1, Comparative Example 2, and Embodiments 1, 2, and 3, respectively, before being placed in the frozen preservation environment (D0) and after 1 week (W1), 2 weeks (W2), 3 weeks (W3), and 4 weeks (W4) of preservation in the frozen environment. The residual concentrations of the extracellular vesicles in each Comparative Example and Embodiment in the frozen environment are compared to evaluate the effectiveness of preserving the extracellular vesicles in the frozen environment for each Embodiment.

FIG. 2 shows a significant degradation of the extracellular vesicles in Comparative Example 1 and Comparative Example 2 even before freezing, while Embodiments 1, 2 and 3 showed excellent cryopreservation effects. In particular, after 4 weeks (W4), Embodiment 1 still maintains the concentrations of the extracellular vesicles at over 85% of the original, while Embodiments 2 and 3 maintain the concentrations between 80% and 85%.

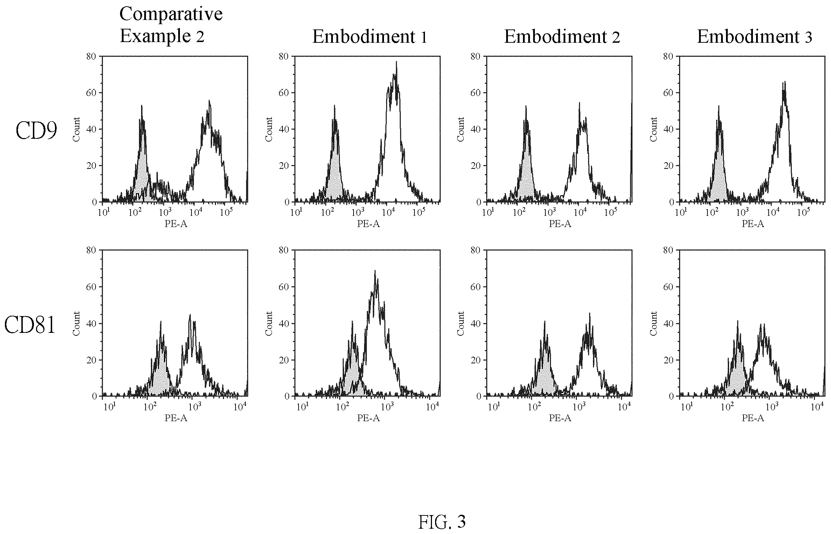

Next, please refer to FIG. 3, where the specific marker proteins CD9 and CD81 on the known extracellular vesicles are separately linked to a developing marker, and the surviving extracellular vesicles after 4 weeks of frozen preservation as per Comparative Example 2 and each Embodiment are tested to verify if they still exhibit biological activity. This serves to validate the applicability of the extracellular vesicles after cryopreservation in Embodiments 1, 2, and 3. The results of the CD9 detection in Embodiments 1, 2, and 3 show significantly intact, confirming that cryopreservation in Embodiments 1, 2, and 3 does not affect the biological activity of the extracellular vesicles, thus facilitating subsequent use.

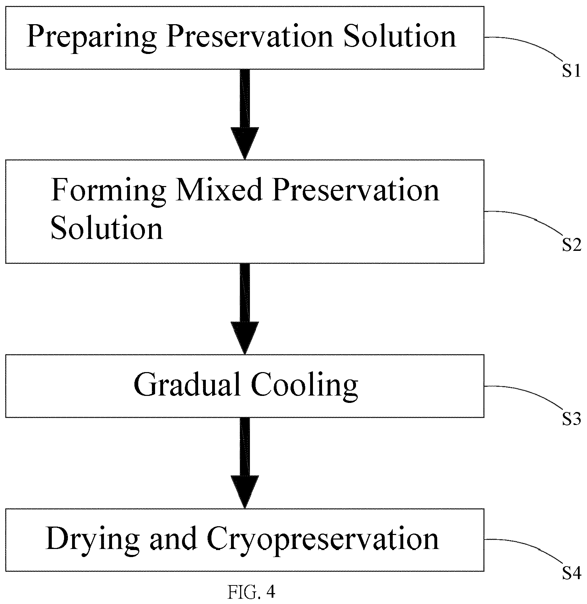

In Experiment 2, referring to FIGS. 4 to 6, wherein the preservation solution is used to carry out the cryopreservation method for the extracellular vesicles/exosomes in the form of dry preservation. This differs from the Experiment 1 above in that the mixed preservation solution is formed and a step S4 is carried out after gradual cooling, followed by drying and cryopreservation. The mixed preservation solution is dried to produce a dried preservation sample, which is then preserved in an environment at -80 degrees Celsius.

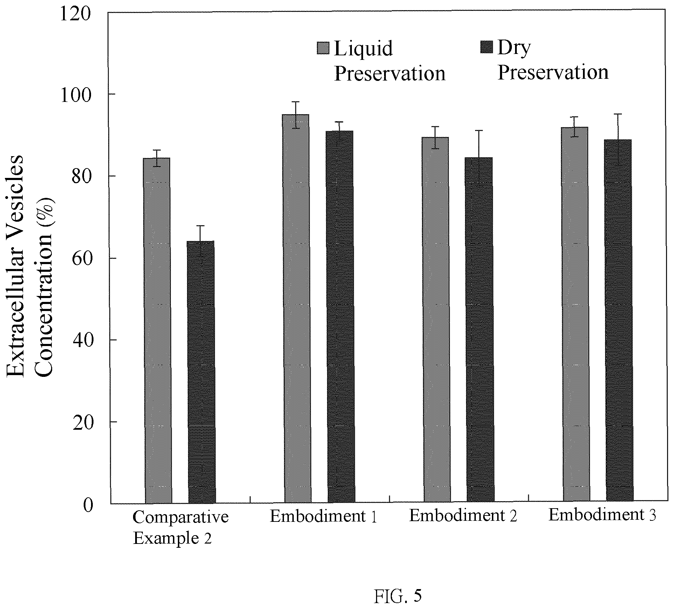

Next, please refer to FIG. 5. The present invention further compares the residual concentrations of the mixed preservation solution and the dried preservation sample in the frozen environment at -80 degrees Celsius for 4 weeks in Comparative Example 2 and each Embodiment. The residual concentration of the extracellular vesicles is compared to evaluate the effectiveness of the cryopreservation methods for preserving the extracellular vesicles in liquid and dry forms in each Embodiment.

In Comparative Example 2, it can be observed that the extracellular vesicles undergo significant degradation after dry preservation using DMSO compared to liquid preservation. However, using the preservation solution provided in Embodiments 1 to 3 for cryopreservation shows excellent preservation effects on the extracellular vesicles. It is also noteworthy that there is no significant difference in the preservation effects of the extracellular vesicles between the liquid and dry preservation methods.

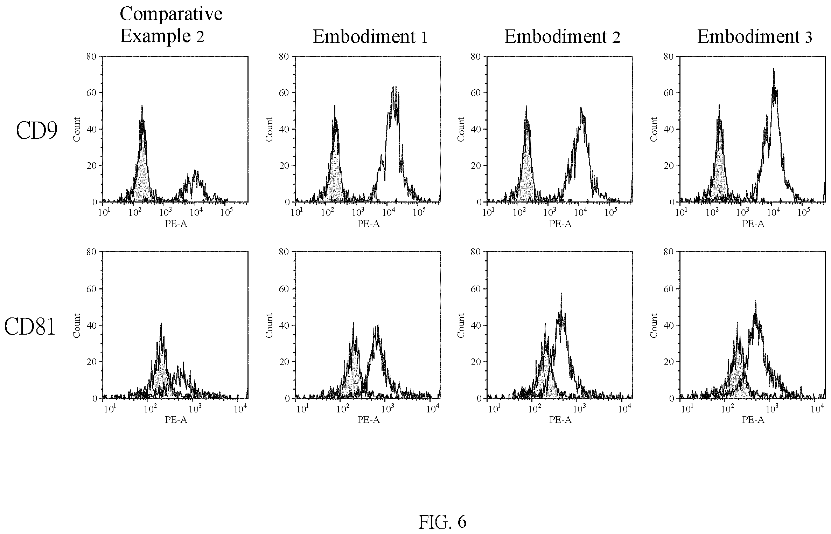

Similarly, in FIG. 6, the marker proteins CD9 and CD81 on the extracellular vesicles are separately linked to the developing marker, and the surviving extracellular vesicles are tested after 4 weeks of dry preservation as per Comparative Example 2 and each Embodiment to verify if they still exhibit biological activity. This serves to validate the applicability of the extracellular vesicles after dry cryopreservation in Embodiments 1, 2 and 3. The results of CD9 detection in Embodiments 1, 2, and 3 show significantly intact, confirming that dry cryopreservation in Embodiments 1, 2, and 3 does not affect the biological activity of the extracellular vesicles, consistent with the above experimental results.

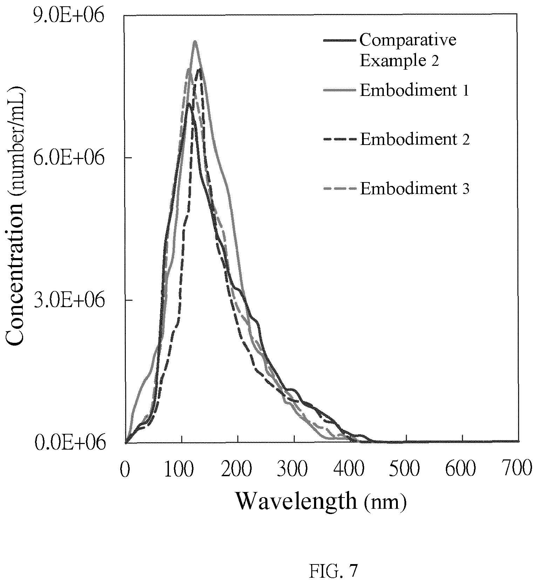

Furthermore, in FIG. 7, a comparative analysis of the size of the extracellular vesicles after dry preservation is performed between Comparative Example 2 and Embodiments 1, 2, and 3. This ensures that the physiological structure of the extracellular vesicles is not affected by the preservation solution and cryopreservation method. As observed in the Figure, after cryopreservation of both Comparative Example 2 and each Embodiment, there is no significant difference in size between the extracellular vesicles. However, the concentration of extracellular vesicles decreases significantly after dry cryopreservation in Comparative Example 2.

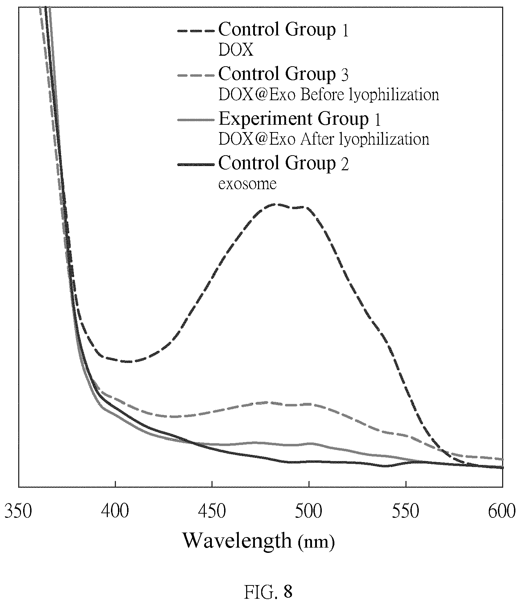

In Experiment 3, referring FIG. 8, the present invention then subjected the extracellular vesicles to a drug-carrying process after thawing. By drug testing to confirm that the extracellular vesicles still possess the functionality to carry the drug after dry cryopreservation via the preservation solution.

In this embodiment, Doxorubicin (DOX) is used as the drug. The drug-carrying efficiency was evaluated by detecting the reflected wavelength of the drug to verify whether the extracellular vesicles were carrying the drug. A better drug-carrying efficiency indicates a higher proportion of drug carried by the extracellular vesicles. The extracellular vesicles with the drug showed more distinct reflected wavelengths between 400 and 550 nm, indicating a higher efficiency in carrying the drug.

In the drug-carrying process provided by the present invention, a cell count of 1011 extracellular vesicles is initially added to the solution having a concentration of 2 mg/mL of Doxorubicin (DOX) and allowed to stand and react. This results in the extracellular vesicles carrying the drug. The uncoated DOX is then removed by centrifugation, leaving the extracellular vesicles ready for further testing.

In this experiment, different groups are established: Control Group 1 with only the drug, Control Group 2 with the extracellular vesicles without drug carrying, Control Group 3 with the drug-carried extracellular vesicles, and Experiment Group 1 with the drug-carried extracellular vesicles subjected to cryopreservation in the form of dry preservation. Wherein, Control Group 3 uses the formula provided in Embodiment 1 as the medium for drug carrying of the extracellular vesicles. Similarly, Experiment Group 1 uses the formula provided in Embodiment 1 as the preservation solution to perform the drug-carrying process prior to cryopreservation. The results show that the drug-carried extracellular vesicles have a lower drug content after dry preservation.

To demonstrate the effectiveness of Experiment Group 1 after the drug-carrying process, the cryopreservation, and the subsequent reconstitution, the present invention further conducted a test to evaluate the therapeutic effectiveness of the extracellular vesicles carrying the drug via the cryopreservation with the preservation solution.

In Experiment 4, the extracellular vesicles are first subjected to the drug-carrying process and then frozen with the preservation solution (dry preservation form). After thawing, the drug-carried extracellular vesicles (Doxorubicin) are tested for cytotoxicity against human breast cancer cells to verify the killing effect of the drug-carried extracellular vesicles.

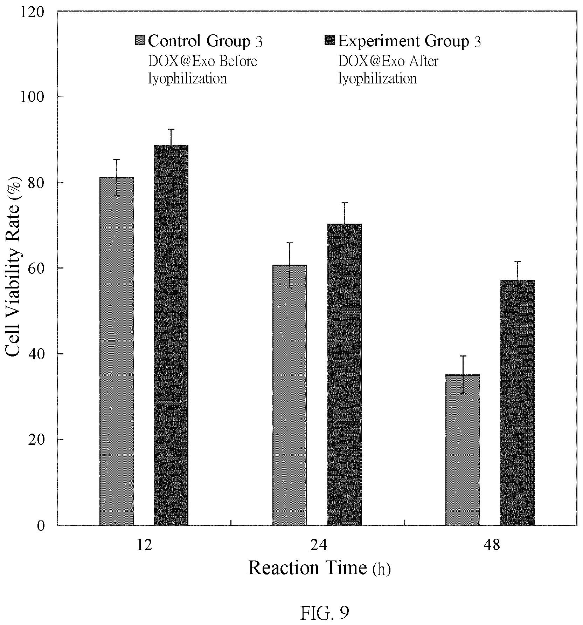

Referring to FIG. 9, the drug-carried extracellular vesicles (Control Group 3) and the drug-carried extracellular vesicles after dry cryopreservation (Experiment Group 1) are separately added to the culture medium of the human breast cancer cells for reaction. Then, the cell viability rate (%) is then measured at 12 hours, 24 hours, and 48 hours before and after the reaction to compare the cytotoxic effects of Control Group 3 and Experiment Group 1. The results indicated that the cytotoxic effect of the drug-carried extracellular vesicles after cryopreservation is lower than that of the group without cryopreservation, but it still possessed therapeutic efficacy in drug delivery.

In Experiment 5, the extracellular vesicles are taken out after cryopreservation (dry preservation form) and subjected to the drug-carrying process after thawing. Likewise, the cytotoxicity test against the human breast cancer cells is conducted to verify the killing effect of the drug-carried extracellular vesicles (Doxorubicin).

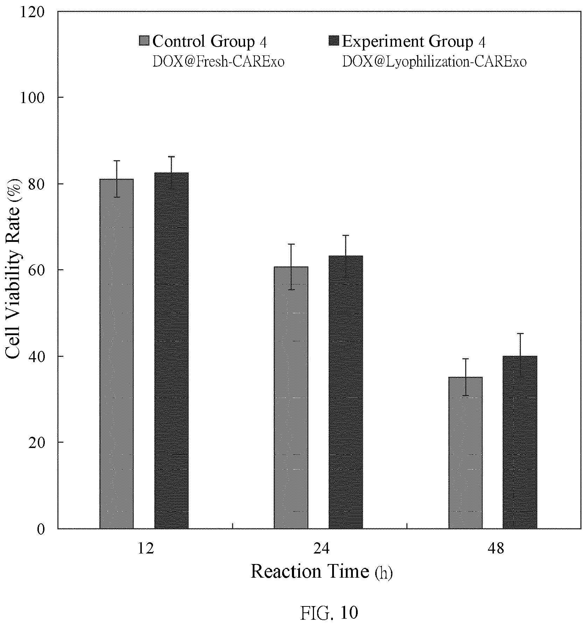

In FIG. 10, the present invention first uses the group in which the extracellular vesicles previously preserved with cryopreservation in the form of dry preservation are thawed and then combined with the drug (DOX) as the Experiment Group 4, the group in which the extracellular vesicles are not subjected to cryopreservation and combined with the drug as the Control Group 4, and both groups are subjected to the cytotoxicity test against the human breast cancer cells. The results indicate that there is no significant difference in the cytotoxic effect against the human breast cancer cells between the groups where the extracellular vesicles are preserved with cryopreservation and then combined with the drug after thawing (Experimental Group 4) and Control Group 4. Both groups exhibit excellent cytotoxic effects.

It is noteworthy that compared to the results in FIG. 9, an optimal procedure for preserving the extracellular vesicles using the preservation solution provided in the present invention and performing the drug carrying can be established.

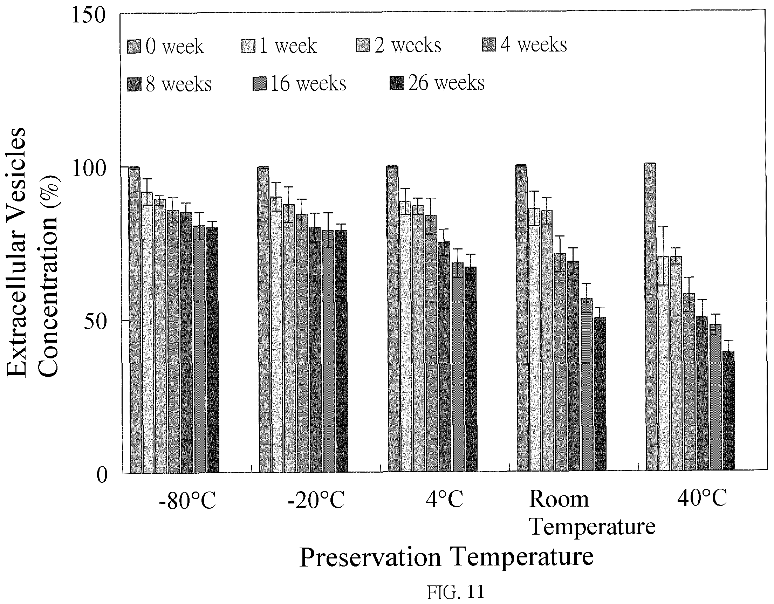

In Experiment 6, as shown in FIG. 11, the preservation solution provided by the present invention is further used to compare the preservation effects of the extracellular vesicles in different preservation environments and to verify the temporal effectiveness of the preservation. The extracellular vesicles are placed in the preservation solution and preserved in different temperature environments, including -80 degrees Celsius, -20 degrees Celsius, 4 degrees Celsius, room temperature, and 40 degrees Celsius. The concentrations of the extracellular vesicles prior to preservation are used as a reference, and the residual concentration percentages of the extracellular vesicles are evaluated for each group after 1, 2, 4, 8, 16, and 26 weeks of preservation.

The results showed the excellent preservation effects of the extracellular vesicles in the freezing environment (at -80°C and -20°C) using the provided preservation solution. The preservation effect could even be extended to over six months (26 weeks), with the residual concentration of the extracellular vesicles reaching 80%. As the preservation temperature increased, the preservation effect of the extracellular vesicles decreased with longer preservation times.

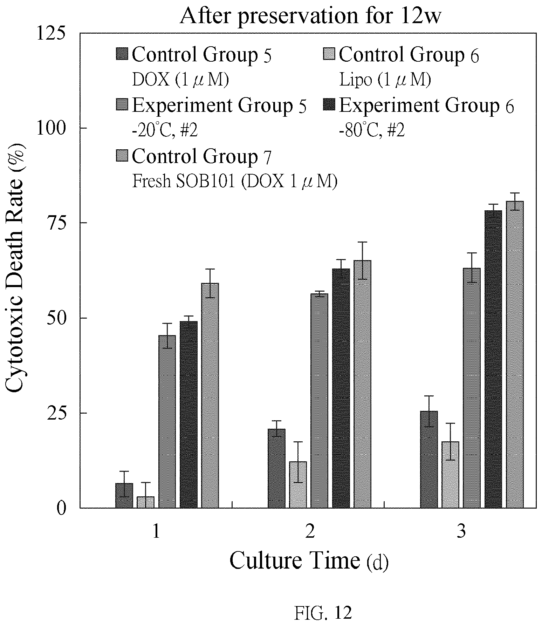

FIGS. 11 and 12 further demonstrate the drug (Doxorubicin) carrying process of the extracellular vesicles preserved in the freezing environment for 12 and 14 weeks using the provided preservation solution. The cytotoxicity tests are also conducted against the human breast cancer cells to verify the efficiency of carrying the drug over an extended preservation period and the killing effect of the extracellular vesicles.

Wherein, the group which directly treats the human breast cancer cells with the drug serves as Control Group 5; the group which treats the human breast cancer cells with the extracellular vesicles without carrying the drug serves as Control Group 6; and the group which does not undergo cryopreservation and carries the drug in the extracellular vesicles serves as Control Group 7. The group of extracellular vesicles carried with the drug after cryopreservation at -20 degrees Celsius serves as Experiment Group 5, while those carried with the drug after cryopreservation at -80 degrees Celsius serves as Experiment Group 6.

The human breast cancer cells are co-cultured with each group for the cytotoxicity test. The cell counts are measured on days 1, 2, and 3 and compared to the total cell count of the human breast cancer cells prior to the cytotoxicity test to calculate the cytotoxic death rate (%).

The results showed a significant cytotoxic effect in Experiment Groups 5 and 6 on the day 1. With increasing co-culture time, the cytotoxic death rate (%) of the human breast cancer cells in Experiment Groups 5 and 6 continues to increase. On the days 2 and 3, Experiment Group 6 exhibits cytotoxic results similar to Control Group 7, demonstrating that the preservation solution provided by the present invention can not only preserve the extracellular vesicles/exosomes for a longer period of time in the freezing environment, but also maintain the biological activity of the extracellular vesicles/exosomes.

The present invention discloses that the provided preservation solution, utilizing the selected carrier solvent and stabilizer, can be tailored according to the specific type of extracellular vesicles/exosomes to suit different preservation conditions and pH levels and enable long-term preservation under the freezing environments.

Moreover, the preservation solution can be used to preserve the extracellular vesicles/exosomes in various preservation environments, which contributes to the commercial development of the product.

Through the configuration of the preservation solution, the present invention allows for cryopreservation in liquid or dry form according to specific needs. This allows for long-term preservation of the extracellular vesicles/exosomes while still maintaining the certain reaction efficiency upon thawing for applications, contributing to research and applications in the medical industry while enhancing time efficiency and commercial value.

## Claims

1. A solution for preserving extracellular vesicles/exosomes, **characterized in that** the solution comprising:
a carrier solvent selected from the group consisting of polysorbate 80, sucrose, and polyethylene glycol 3350/4000 (PEG-3350/4000); and
a stabilizer selected from the group consisting of salts, amino acids, and amino acid salts; wherein
the pH of the preservation solution is in the range of 5 to 7.4.

2. The preservation solution according to claim 1, **characterized in that** the salt is a sodium acetate or a sodium chloride.

3. The preservation solution according to claim 1, **characterized in that** the amino acids are selected from the group consisting of histidine, arginine, and glycine, and the weight percent concentration of the amino acid is in the range of 0.5 to 3 of the total volume of the preservation solution.

4. The preservation solution according to claim 1, **characterized in that** the amino acid salts are selected from the group consisting of histidine hydrochloride, arginine hydrochloride, and glycine hydrochloride, and the weight percent concentration of the amino acid is in the range of 0.05 to 0.25 of the total volume of the preservation solution.

5. The preservation solution according to any one of claims 1 to 4, **characterized in that** the weight percent concentration of polysorbate 80 is in the range of 0.01 to 0.03 of the total volume of the preservation solution, and the weight percent concentration of sucrose is in the range of 5 to 10 of the total volume of the preservation solution.

6. A method for preserving extracellular vesicles/exosomes, **characterized in that** any one of the preservation solutions according to claims 1 to 5, the steps including:
adding the extracellular vesicles/exosomes to the preservation solution to form a mixed preservation solution; and
gradually cooling the mixed preservation solution and placing the mixed preservation solution in a frozen preservation environment for preservation.

7. The preservation method according to claim 6, **characterized in that** the temperature of the frozen preservation environment is -20 degrees Celsius or -80 degrees Celsius.

8. The preservation method according to claim 6 or 7, **characterized in that** after forming the mixed preservation solution, first drying to form a dried preservation sample, and then placing the dried preservation sample in the frozen preservation environment for preservation.

9. A mixed solution **characterized in that** the mixed solution comprising any one of the preservation solutions according to claims 1 to 5 and the extracellular vesicles/exosomes.

10. The mixed solution according to claim 9, **characterized in that** the extracellular vesicles/exosomes in the mixed preservation solution are in the range of 10¹¹ to 10¹³ cells per milliliter.

11. The mixed solution according to claim 9, **characterized in that** the extracellular vesicles/exosomes are modified extracellular vesicles carrying a specific protein.

12. The mixed solution according to any one of claims 9 to 11, **characterized in that** the extracellular vesicles/exosomes are carried with a drug.
